(19) Europäisches Patentamt
European Patent Office
Office européen des brevets

(11) EP 1 388 347 B1

(12) **FASCICULE DE BREVET EUROPEEN**

(45) Date de publication et mention de la délivrance du brevet:
**25.07.2007 Bulletin 2007/30**

(21) Numéro de dépôt: **03291859.1**

(22) Date de dépôt: **28.07.2003**

(51) Int Cl.:
*A61L 31/16* (2006.01) *A61L 31/14* (2006.01)
*A61L 31/04* (2006.01) *A61F 6/04* (2006.01)
*A61B 19/04* (2006.01) *A61L 15/24* (2006.01)

(54) **Matériau élastomère multicouches chargé en substance chimique active et ses utilisations**

Merschichtiges Elastomermaterial mit einem chemischen Wirkstoff und dessen Anwendungen

Multilayered elastomer material containing a chemical active substance and applications thereof

(84) Etats contractants désignés:
**AT BE BG CH CY CZ DE DK EE ES FI FR GB GR HU IE IT LI LU MC NL PT RO SE SI SK TR**

(30) Priorité: **31.07.2002 FR 0209734**

(43) Date de publication de la demande:
**11.02.2004 Bulletin 2004/07**

(73) Titulaire: **HUTCHINSON**
**75008 Paris (FR)**

(72) Inventeurs:
- **Hoerner, Pierre**
  **60660 Maysel (FR)**
- **Sonntag, Philippe**
  **77850 Hericy (FR)**
- **Cheymol, André**
  **86220 Dange Saint Romain (FR)**
- **Krikorian, Raffi**
  **95290 L'Isle Adam (FR)**

(74) Mandataire: **Goulard, Sophie et al**
**Cabinet ORES**
**36, rue de St Pétersbourg**
**75008 Paris Cedex (FR)**

(56) Documents cités:
**WO-A-99/47589**
**US-A- 5 338 565**
**US-A- 5 804 628**
**US-A- 5 965 276**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 388 347 B1

Printed by Jouve, 75001 PARIS (FR)

## Description

La présente Invention est relative à un matériau multicouches réalisé à partir d'élastomères et comprenant au moins une couche contenant une ou plusieurs substances actives dispersées sous forme de gouttelettes, ainsi qu'à ses utilisations, notamment à titre de gant, de doigtier ou de préservatifs.

Les différents matériaux élastomères, habituellement utilisés dans le domaine médical ou paramédical (hygiène, notamment) peuvent être modifiés, de manière à être associés à des substances chimiques actives, ayant un effet de protection, lors de l'utilisation de ce matériau (gants, doigtiers, préservatifs, bandes et pansements divers). En effet, aussi bien dans les cas d'examen ou d'intervention chirurgicale ou en odontologie, que pour la protection à l'encontre d'agents pathogènes, tels que, par exemple bactéries, virus et spores fongiques, une rupture ou même parfois simplement les pores ou une fissure de la membrane d'élastomère, peut entraîner une contamination du porteur dudit matériau par piqûres de seringues, d'aiguilles à suture, de trocart, d'éclats d'os, etc...

C'est ainsi qu'il a déjà été proposé, notamment dans la demande de brevet EP-A-0 981 573, des films d'élastomères dans lesquels sont dispersées uniformément, sous forme de gouttelettes de liquides, des substances chimiques actives telles que des agents désinfectants à usage médical. De tels matériaux se présentent généralement sous la forme de trois couches distinctes, $L_1$, $L_2$ et $L_3$ telles que représentées sur le schéma I ci-dessous :

**SCHÉMA I**

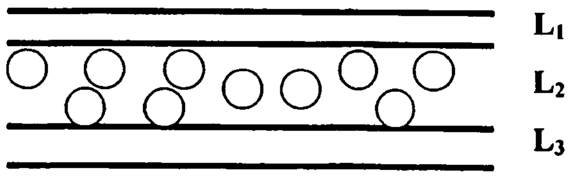


dans lequel les couches $L_1$ et $L_3$ sont des couches barrières externes et $L_2$ représente une couche intermédiaire contenant la ou les substances chimiques actives dispersées sous la forme de gouttelettes.

Dans ces matériaux, le liquide contenant la ou les substances chimiques actives est dispersé de manière uniforme et stable sous forme de gouttelettes et est ainsi disponible en cas de rupture accidentelle de la couche externe protectrice $L_1$ ou $L_3$.

Malheureusement, de tels films ne présentent pas toujours les performances souhaitées. D'une part, la quantité de substances chimiques actives entrant en contact avec l'objet acéré est bien souvent insuffisante pour assurer les propriétés désirées, d'autre part, le fluide est seulement mis en contact et non expulsé sur l'objet contondant, n'assurant ainsi qu'un mouillage partiel de celui-ci, ce qui entraîne une faible efficacité de ce type de dispositifs. Ce dernier point est d'autant plus important que la géométrie de l'objet acéré est souvent complexe (aiguilles creuses, biseautées, etc...).

C'est donc afin de remédier à ces problèmes majeurs que, poursuivant ses recherches, la Demanderesse a découvert que, de manière surprenante, les caractéristiques finales du matériau multicouches, telles que la quantité de substance active déposée sur l'objet contondant et la vitesse d'expulsion des gouttelettes de substance active, sont fonction de la combinaison des caractéristiques intrinsèques de chacune des couches constitutives du matériau.

Selon la présente Invention, on entend par "caractéristiques intrinsèques", les caractéristiques mécaniques (contrainte à la rupture et constante élastique) et dimensionnelles (épaisseur) des élastomères formulés constituant chaque couche, ainsi que, pour la couche intermédiaire $L_2$, le taux de remplissage et la taille des gouttelettes. Dans cette Invention les caractéristiques intrinsèques de chacune des couches sont ajustées par considération au matériau multicouches pris dans sa globalité.

Les Inventeurs se sont donc fixés pour but de pourvoir à un matériau élastomère multicouches renfermant au moins une substance active, notamment apte à être utilisé dans le domaine médical ou paramédical pour la fabrication de dispositifs de protection présentant des performances améliorées en ce qui concerne la quantité de substance active entrant en contact avec un objet contondant en cas de perforation dudit dispositif ainsi qu'en ce qui concerne la vitesse à laquelle cette substance active entre en contact avec ledit objet contondant par rapport aux matériaux similaires de l'art antérieur.

L'invention a donc pour premier objet un matériau élastomère multicouches comportant au moins deux couches barrières externes $L_1$ et $L_3$, présentant respectivement une contrainte à la rupture $\sigma_1$ et $\sigma_3$ et une épaisseur $e_1$ et $e_3$, emprisonnant au moins une couche intermédiaire $L_2$ constituée par une matrice d'élastomère comprenant au moins une dispersion de gouttelettes d'au moins une composition renfermant au moins une substance active, ladite couche intermédiaire $L_2$ ayant une contrainte à la rupture $\sigma_{2Tot}$ et une épaisseur $e_2$, caractérisé par le fait que le diamètre moyen desdites gouttelettes est supérieur ou égal à 10 $\mu$m et que ledit matériau remplit la double inégalité (I) suivante :

$$(\sigma_{2Tot}.e_2) < (\sigma_1.e_1) \text{ et } (\sigma_{2Tot}.e_2) < (\sigma_3.e_3) \qquad (I)$$

inégalité dans laquelle :

- $\sigma_{2Tot}$ représente la contrainte à la rupture du matériau élastomère chargé constitutif de la couche $L_2$,
- $\sigma_1$, $\sigma_3$, $e_1$, $e_2$ et $e_3$ sont tels que définis ci-dessus.

Dans les inégalités définies ci-dessus, $\sigma_{2Tot}$ est fonction de la fraction volumique $\phi$v des gouttelettes incorporées au sein de la couche $L_2$, et des caractéristiques intrinsèques du matériau élastomère constituant la matrice ($\sigma_2$). A cet égard, $\phi$v peut bien entendu à ajustée pour satisfaire aux inégalités ci-dessus concernant la contrainte à la rupture $\sigma_{2Tot}$.

Grâce à ces caractéristiques particulières, et lorsqu'une pression est exercée sur le matériau conforme à l'Invention avec un objet acéré, la couche intermédiaire $L_2$ cède avant les couches externes $L_1$ et $L_3$ et les gouttelettes sont expulsées sur l'objet acéré après la rupture de la couche barrière externe ($L_1$ et/ou $L_3$).

Les Inventeurs ont en effet mis en évidence que la rupture des couches constitutives d'un matériau multicouches est essentiellement fonction de la résistance à l'effort tranchant ou perçant de chacune des couches qui est proportionnelle au produit de la contrainte à la rupture multipliée par l'épaisseur pour chacune des couches. La résistance à la rupture de la couche intermédiaire est de plus fonction de la quantité de composition renfermant la substance active comprise dans cette couche (fraction volumique de liquide $\phi$v).

Selon l'Invention, les contraintes à la rupture $\sigma_1$, $\sigma_2$, $\sigma_{2Tot}$ et $\sigma_3$ sont mesurées expérimentalement au moyen d'un dynamomètre Zwick, à une vitesse de 500 mm/min et avec des éprouvettes H2 selon la norme NF EN455-2.

Selon une forme de réalisation avantageuse de l'Invention, le produit ($\sigma_{2Tot}.e_2$) répond à la double condition (II) suivante :

$$(\sigma_{2Tot}.e_2) \leq (\sigma_1.e_1)/2 \text{ et } (\sigma_{2Tot}.e_2) \leq (\sigma_3.e_3)/2 \qquad (II)$$

dans laquelle $\sigma_1$, $\sigma_{2Tot}$, $\sigma_3$, $e_1$, $e_2$ et $e_3$ ont les mêmes significations que celles indiquées ci-dessus.

Les contraintes à la rupture $\sigma_1$, $\sigma_2$ et $\sigma_3$ de chacune des couches du matériau conforme à l'Invention, identiques ou différentes, varient de préférence entre 0,1 et 100 MPa, et encore plus préférentiellement entre 5 et 40 MPa.

Les épaisseurs $e_1$, $e_2$ et $e_3$ de chacune des couches du matériau conforme à l'Invention, identiques ou différentes, varient de préférence entre 25 et 500 $\mu$m environ, cette épaisseur étant choisie en fonction de la destination d'usage dudit matériau.

A titre d'exemples non limitatifs, lorsque le matériau conforme à l'Invention est destiné à être utilisé pour la fabrication de préservatifs, alors l'épaisseur de chacune des couches est d'environ 25 $\mu$m; lorsqu'il est utilisé pour la fabrication de gants de chirurgie, alors l'épaisseur de chacune des couches est d'environ 100 $\mu$m et lorsqu'il est utilisé pour la fabrication de gants de protection par exemple de gants de ménage ou de gants pour éboueurs, alors l'épaisseur de chacune des couches est d'environ de 500 $\mu$m.

Selon une forme de réalisation préférée du matériau conforme à l'invention, le diamètre moyen des gouttelettes dispersées au sein de la couche $L_2$ est de préférence compris entre 10 et 100 $\mu$m environ.

De manière préférentielle, les Inventeurs ont également mis en évidence que l'expulsion de la composition renfermant la substance active sur un objet contondant venant perforer l'une des deux couches barrières $L_1$ ou $L_3$ est encore améliorée en fonction de la viscoélasticité des matériaux constitutifs de chaque couche, et plus particulièrement de leur constante élastique (E), soit $E_1$, $E_2$ et $E_3$ respectivement.

Ainsi, selon un mode de réalisation avantageux de l'Invention, la constante élastique du matériau constitutif de la couche intermédiaire $L_2$ est supérieure à celles des couches barrières $L_1$ et $L_3$ ($E_1$ et $E_3$) soit $E_2 > \max(E_1, E_3)$.

Selon l'Invention, les constantes élastiques de chacune des couches $L_1$, $L_2$ et $L_3$, soit respectivement $E_1$, $E_2$ et $E_3$, sont de préférence comprises entre 0,1 et 50 MPa, et encore plus préférentiellement entre 0,1 et 10 MPa pour les couches $L_1$ et $L_3$ et entre 0,5 et 50 MPa pour la couche $L_2$; les valeurs de $E_1$ et de $E_3$ étant identiques ou différentes.

Selon l'Invention, les constantes élastiques sont mesurées au moyen d'un viscoanalyseur de type DMTA (Dynamical Thermal Analysis tel que l'appareil VA2000 de la société METRAVIB), capable d'évaluer, dans des gammes de températures usuelles, les caractéristiques viscoélastiques des matériaux considérés, à une fréquence de 1 Hz (sollicitation dans le domaine viscoélastique linéaire), en mode traction-compression, selon la méthode décrite dans la norme NF EN ISO 527-3.

Selon l'Invention, le ou les élastomères constituant les couches barrières externes $L_1$ et $L_3$ ainsi que la couche intermédiaire $L_2$, sont de préférence choisis parmi le caoutchouc naturel, le polybutadiène, le polyisoprène, le polychloroprène, le polyuréthane, les copolymères ou polymères acryliques, les élastomères silicones, les copolymères SBR

*(Styrène Butadiene Rubber),* SBS *(Styrène Butadiene Styrene),* isobutène-isoprène tels que le caoutchouc butyle, NBR *(Nitrite Butadiene Rubber),* x-NBR (carboxylated Nitrile Butadiene Rubber), SIS *(Styrene Isoprene Styrene),* SEBS *(Styrène Ethylene Butylène Styrene)* et leurs mélanges ; étant entendu que la nature du ou des élastomères constituant chacune desdites couches peut être identique ou différente d'une couche à l'autre. Selon l'Invention, le SIS et le SEBS sont préférés.

Les caractéristiques des élastomères (masse molaire, densité de réticulation (chimique et/ou physique) sont sélectionnées en fonction des caractéristiques finales souhaitées pour les couches $L_1$ et $L_3$ et pour la couche $L_2$, sous réserve qu'elles répondent, bien entendu, aux définitions données ci-dessus.

En plus des élastomères définis ci-dessus, l'une au moins des couches barrières $L_1$ et $L_3$, et/ou la couche intermédiaire $L_2$, peut en outre contenir un ou plusieurs agent(s) plastifiants) ou flexibilisant(s) dont la nature chimique et la teneur sont compatibles avec les caractéristiques intrinsèques du matériau telles que définies précédemment.

Lorsqu'ils sont utilisés, ces agents plastifiants sont de préférence choisis parmi les huiles minérales parmi lesquelles on peut notamment citer les huiles paraffiniques, mais peuvent également être choisis parmi les huiles naphtaléniques ou aromatiques ou les mélanges de ces produits.

Lorsqu'ils sont utilisés, le ou les agents plastifiants) représentent de préférence de 5 à 500 parts pour 100 parts d'élastomère constituant la couche au sein de laquelle ils sont présents.

Il est à noter que la nature chimique, la composition ou l'épaisseur de la couche $L_1$ n'est pas nécessairement équivalente à celle de la couche $L_3$.

Enfin, chaque couche $L_1$ ou $L_3$ peut, en variante, résulter de la superposition de deux ou plusieurs sous-couches de nature chimique équivalentes ou non. Les caractéristiques intrinsèques de la couche $L_1$ ou $L_3$ seront alors celles mesurées sur la couche complète.

La couche intermédiaire $L_2$ sert de matrice aux gouttelettes de composition renfermant la ou les substances chimiques actives utilisées.

Selon l'Invention, la nature de cette substance active pourra être choisie en fonction des propriétés que l'on souhaite conférer à la couche intermédiaire $L_2$. Cette substance chimique active peut notamment être choisie parmi les agents anti-corrosion, les lubrifiants, les marqueurs chimiques, les produits à changement de phase, les ralentisseurs de particules énergétiques (radiation), les agents possédant un pouvoir désinfectant, les agents odorants ou hydratants, les colorants détecteurs de coupures, les particules métalliques, et leurs mélanges.

Lorsque la substance chimique active est un produit possédant un pouvoir désinfectant, celui-ci est de préférence choisi parmi les substances capables de provoquer une dénaturation quasi-instantanée des protéines par simple contact, soit par réaction chimique, soit par un effet physico-chimique tel qu'une modification de la tension de surface. Parmi de telles substances on peut notamment citer les biocides, tels que les ammoniums quaternaires et plus particulièrement le chlorure de diméthyldidécylammonium et le chlorure de benzalkonium, les biguanides tels que les sels hydrosolubles de la chlorhexidine comme le digluconate de chlorhexidine, le phtalaldéhyde, les dérivés phénoliques tels que l'hexachlorophène ou benzyliques, le formol, les tensioactifs nonioniques comportant au moins une séquence polyoxyéthylène tel que l'octoxynol (ou Triton® X100), l'hexamidine, les composés iodés de polyvinylpyrrolidone, les tensioactifs nonioniques à activité virucide, les bichromates et hypochlorites de sodium et de potassium, et leurs mélanges.

En plus de la ou des substances chimiques actives, la composition sous forme de gouttelettes peut en outre renfermer un ou plusieurs diluants permettant de solubiliser ladite ou lesdites substances chimiques actives.

Lorsqu'il est utilisé, ce diluant peut notamment être choisi parmi les polyols et de préférence parmi la glycérine, l'éthylène glycol, les polyéthylènes glycols liquides à température ambiante ou à une température voisine de la température ambiante (entre 20 et 30°C environ) et de masse molaire comprise entre 62 (éthylène glycol) et 750 Daltons (polyéthylèneglycol : PEG 750) et leurs mélanges, ainsi que parmi tout autre composé compatible avec la ou les substances chimiques actives utilisées.

En outre, la composition sous forme de gouttelettes peut également contenir un ou plusieurs additifs permettant d'ajuster les caractéristiques finales du mélange tels que par exemple des colorants, des agents tensioactifs ou des agents épaississants.

La composition sous forme de gouttelettes et renfermant la substance active peut être sous forme liquide (sous la forme d'une émulsion), gélifiée ou contenir des parties cristallines (microsphères). Lorsque cette composition se présente sous la forme de microsphères, celles-ci peuvent en outre être enrobées d'une fine pellicule de polymère protecteur (microcapsules).

Selon une forme de réalisation particulière de l'Invention, la couche intermédiaire $L_2$ peut être formée d'une superposition de deux ou plusieurs sous-couches intermédiaires comprenant chacune une dispersion de gouttelettes, la nature des substances actives contenues dans chacune desdites sous-couches étant identiques ou différentes d'une sous-couche à une autre. Dans ce cas, les caractéristiques intrinsèques de la couche $L_2$ seront alors celles mesurées sur la couche complète.

Selon une autre forme de réalisation particulière de l'Invention, la couche intermédiaire $L_2$ est formée par une

seule couche renfermant une dispersion de gouttelettes contenant des substances chimiques actives différentes d'une gouttelette à une autre.

Chacune des couches constituant le matériau élastomère conforme à l'Invention peut contenir, en outre, d'autres adjuvants classiquement employés dans l'industrie des polymères comme par exemple des antistatiques, des lubrifiants, des antioxydants, des colorants, des agents de mise en oeuvre ou encore des promoteurs d'adhérence selon les propriétés particulières que l'on souhaite lui donner pour autant, bien entendu, que ces adjuvants soient compatibles entre eux et avec les caractéristiques intrinsèques dudit matériau telles que définies précédemment.

Selon une variante de l'Invention, le matériau multicouches peut être renforcé par une trame textile élastique de fibres organiques naturelles ou synthétiques servant alors de support pour l'une des deux couches barrières $L_1$ ou $L_3$. Lorsque la trame textile est adjacente à la couche barrière $L_1$, ce type de matériau multicouches peut être représenté par le schéma II ci-après :

**SCHEMA II**

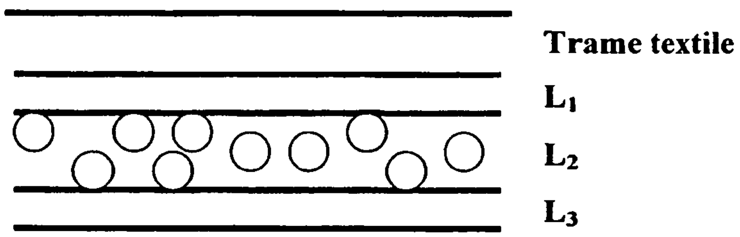


Le lien entre les différentes couches constitutives du matériau conforme à l'Invention peut, optionnellement, être assuré par un agent de collage ou par une modification chimique ou physico-chimique de l'une quelconque des couches. Un tel traitement n'a cependant pas d'influence sur les caractéristiques finales du matériau.

Selon l'Invention, on entend par modification chimique soit un greffage, soit une attaque chimique, et par modification physico-chimique un bombardement de la surface du film avec des ions, des électrons ou des photons.

Le matériau élastomère multicouches conforme à l'Invention peut notamment se présenter sous la forme de gants, doigtiers, préservatifs, bandes, pansements, etc.....

La présente Invention a donc également pour objet l'utilisation d'au moins un matériau élastomère multicouches tel que défini ci-dessus pour la fabrication d'articles élastomères de protection tels que gants, doigtiers, préservatifs, bandes ou pansements.

La fabrication du matériau multicouches tel que défini ci-dessus, peut être réalisée selon un procédé de trempes et d'évaporations successives d'une forme correspondant à l'utilisation envisagée pour ledit matériau, dans des solutions organiques ou des dispersions aqueuses (latex) du ou des élastomères choisis afin de former successivement les couches $L_1$, $L_2$ et $L_3$, la formation de la couche $L_2$ étant par exemple réalisée des procédés, consistant :

- soit à préparer une émulsion stable formée par les gouttelettes d'une composition liquide renfermant la ou les substances chimiques actives dans une dissolution de l'élastomère dans un solvant volatile tel que cela est décrit notamment dans la demande de brevet EP-A-0 981 573 ;
- soit à préparer une dispersion desdites gouttelettes sous forme gélifiées, ou cristallisées (microsphères) dans une dissolution de l'élastomère dans un solvant volatile tel que décrit dans la demande de brevet EP-A-771 837. Optionnellement, les microsphères peuvent être enrobées d'une fine pellicule de polymère protecteur (microcapsules) ;
- soit à déposer les gouttelettes, par exemple sous forme de microsphères ou de microcapsules sur la couche $L_1$ et/ou $L_3$, puis à recouvrir lesdites gouttelettes par un élastomère, soit sous forme d'une solution de celui-ci dans un solvant organique, soit sous forme d'une dispersion aqueuse (latex), soit sous forme solide.

Au cours de ce procédé, chaque trempe est suivie d'une période d'évaporation, généralement en étuve thermostatée, au cours de laquelle le solvant ou l'eau, est éliminé.

Lorsque le matériau multicouches conforme à la présente Invention comprend un support en matière textile, alors la première trempe s'effectue avec une forme en porcelaine recouverte d'une trame textile.

Outre les dispositions qui précèdent, l'Invention comprend encore d'autres dispositions qui ressortiront de la description qui va suivre, qui se réfère à des exemples de préparation de matériaux élastomères multicouches conformes à l'Invention, ainsi qu'à un exemple comparatif de préparation d'un matériau multicouches non conforme à l'Invention.

Il doit être bien entendu toutefois que ces exemples sont donnés uniquement à titre d'illustration de l'objet de l'Invention, dont ils ne constituent en aucune manière une limitation.

**EXEMPLE 1 : PRÉPARATION D'UN MATÉRIAU ÉLASTOMÈRE MULTICOUCHE CONFORME À L'INVENTION**

Cet exemple décrit la préparation d'un matériau multicouches réalisé entièrement à partir d'élastomère de synthèse en milieu solvant.

**1) Préparation du matériau multicouches**

On prépare un premier bain d'élastomère dans un solvant (cyclohexane) constitué par 20 % en poids (par rapport à l'extrait sec) d'un mélange de copolymère SEBS vendu sous la dénomination commerciale Kraton® G1652, par la société Kraton Polymers et d'une huile minérale (Primol® 352, société Esso) utilisée à titre d'agent plastifiant. Au sein de ce mélange les proportions massiques élastomère/agent plastifiant sont 100:30. Ce bain est utilisé pour réaliser les couches barrières $L_1$ et $L_3$.

Par ailleurs, et selon le procédé décrit dans la demande de brevet EP-A-981 573, on prépare un bain d'élastomère (SEBS : KRATON® G1652 à 20 % en poids d'extrait sec dans du cyclohexane) renfermant une dispersion de gouttelettes de substance active (chlorure de benzalkonium en solution dans de l'éthylène glycol, en proportions massiques 1:9) stabilisée par un stabilisant particulaire organique qui est un copolymère dibloc polybutadiène-poly(oxyde d'éthylène) à raison de 5 parts pour 100 parts de solution de chlorure de benzalkonium dans l'éthylène glycol.

Au sein de cette dispersion, le diamètre moyen des gouttelettes (mesuré au microscope optique) est de 30 $\mu$m. La fraction volumique des gouttelettes $\phi v$ est de 0,5 (par rapport à l'extrait sec après mélange avec l'élastomère en solution dans le cyclohexane).

Le matériau multicouche est ensuite préparé par trempes successives d'une forme en porcelaine de la façon suivante :

1) formation de la couche barrière $L_1$: deux trempes successives dans le premier bain d'élastomère ;
2) formation de la couche intermédiaire $L_2$ : 1 trois trempes successives dans le second bain d'élastomère contenant la dispersion de la substance active ; puis
3) formation de la couche barrière $L_3$ : deux trempes successives dans le premier bain d'élastomère ;
étant entendu que chaque étape de trempe est immédiatement suivie d'une étape d'évaporation du cyclohexane, d'abord à l'air libre, puis dans une étuve à une température de 40°C, ce jusqu'à évaporation complète du solvant.

On obtient ainsi un matériau multicouches constitué de deux couches barrières $L_1$ et $L_3$ identiques entres elles, d'une épaisseur de 175 $\mu$m chacune et d'une couche intermédiaire $L_2$ d'une épaisseur de 200 $\mu$m, renfermant une dispersion de gouttelettes de chlorure de benzalkonium (les épaisseurs sont mesurées au comparateur micrométrique).

**2) Caractérisation du matériau multicouches**

Afin de déterminer les caractéristiques intrinsèques de chacune des couches constituant ce matériau, on prépare deux films élastomères distincts en réalisant :

- d'une part, la trempe d'une première forme en porcelaine dans le premier bain d'élastomère (selon la technique décrite ci-dessus à l'étape 1) de préparation du matériau multicouches) de façon à réaliser un matériau monocouche uniquement constitué d'une couche $L_1$;
- d'autre part, la trempe d'une seconde forme en porcelaine dans le second bain d'élastomère (selon la technique décrite ci-dessus à l'étape 2) de préparation du matériau multicouches) de façon à réaliser un matériau monocouche uniquement constitué d'une couche $L_2$.

Les couches distinctes $L_1$ et $L_2$ ainsi préparées ont ensuite été étudiées afin de mesurer leurs caractéristiques intrinsèques.

Pour chacune des couches $L_1$ et $L_{2,}$ les contraintes à la rupture ont été mesurées au moyen d'un dynamomètre (Zwick) sur des éprouvettes de type H2, à une vitesse de 500 mm/min, conformément à la norme NF EN455-2.

Les constantes élastiques sont mesurées au moyen d'un viscoanalyseur VA2000 (METRAVIB R.D.S., Limonest, France) sur des éprouvettes rectangulaires des couches $L_1$ et $L_2$, d'une dimension de 20 x 50 mm, en mode traction-compression, conformément à la méthode décrite dans la norme NF EN ISO 527-3.

La quantité de substance chimique active expulsée sur une aiguille lors de la perforation du gant est évaluée sur le matériau multicouches, au moyen d'un test standardisé consistant à :

- couper le matériau multicouche selon un plan de coupe vertical $L_1$, $L_2$, $L_3$, sous azote liquide,
- plaquer le plan de coupe sur une lamelle de verre,

- placer l'ensemble dans le champ d'un microscope à réflexion (Questar),
- piquer le matériau multicouches avec une aiguille creuse d'un diamètre de 0,7 mm, remplie d'eau ultra pure, avec une vitesse de piqûre de 15 cm/s,
- mesurer la vitesse d'expulsion du chlorure de benzalkonium sous le champ du microscope à l'aide d'une caméra vidéo,
- à doser analytiquement la quantité de chlorure de benzalkonium déposée sur l'aiguille au moyen d'un dispositif d'électrophorèse capillaire.

**3) Résultats**

Les caractéristiques du matériau multicouches sont résumées dans le tableau I ci-après :

**Tableau I**

| Couches | Contrainte à la rupture σ (en Mpa) | Constante élastique E (en Mpa) | Produit (σ.e) (en Pa.m) |
|---|---|---|---|
| $L_1$ | 25 | 2 | 4375 |
| $L_2$ | 9 [a] | 7 | 1800 |
| $L_3$ | 25 | 2 | 4375 |
| [a] : la valeur indiquée est la contrainte à la rupture de la couche élastomère chargée en chlorure de benzalkonium. | | | |

La quantité de chlorure de benzalkonium déposée sur l'aiguille est évaluée à 1 µg; la vitesse d'expulsion est estimée à 5 m/s.

**EXEMPLE 2 : PREPARATION D'UN MATERIAU MULTICOUCHES CONFORME A L'INVENTION**

Cet exemple a pour but d'illustrer la préparation d'un matériau "hybride" réalisé à partir de caoutchouc naturel et d'élastomère de synthèse par un procédé mixte eau et solvant.

**1) Préparation du matériau multicouches**

Dans ce cas, ledit matériau est obtenu par trempes successives d'une forme en porcelaine dans une dispersion aqueuse de caoutchouc naturel (latex) pour former les deux couches barrières $L_1$ et $L_3$ et dans une solution d'élastomère de synthèse dans le cyclohexane, ladite solution renfermant une dispersion de substance active pour former la couche $L_2$.

a) Réalisation des couches barrières $L_1$ et $L_3$

Les couches barrières sont formées par une superposition de deux sous-couches.

La première sous-couche est formée par trempe (deux fois) d'une forme en porcelaine dans un bain de latex nitrile (x-NBR : copolymère butadiène-acrylonitrile-acide méthacrylique, vendu par la société Polymer Latex) à 50 % (extrait sec) comprenant également 10 % d'oxyde de zinc, 2 % de diéthyldithiocarbamate de zinc (ZDEC) et 1,5 % de soufre.

La deuxième sous-couche est formée par trempe (une fois) de la forme dans un mélange de latex naturel (NR) et de latex nitrile x-NBR, en proportion massique 75:25. Le latex nitrile est le même que celui utilisé ci-dessus pour former la première sous-couche. Le latex naturel (Heveatex) est formulé avec 1 % de soufre et 0,8 % de ZDEC.

L'extrait sec du mélange latex nitrile/latex naturel est de l'ordre de 45 %.

b) Formation de la couche $L_2$

Elle est réalisée comme décrit ci-dessus à l'exemple 1.

On obtient ainsi un matériau multicouches constitué de deux couches barrières $L_1$ et $L_3$ identiques entres elles, d'une épaisseur de 250 µm chacune et d'une couche intermédiaire $L_2$ d'une épaisseur de 200 µm, renfermant une dispersion de gouttelettes de chlorure de benzalkonium.

Chaque étape de trempe est immédiatement suivie d'une étape d'évaporation de l'eau, d'abord à l'air libre, puis dans une étuve à une température de 40°C (évaporation du cyclohexane pour la couche $L_2$) ou à 60°C (évaporation de l'eau pour les couches $L_1$ et $L_3$), et ce jusqu'à évaporation complète du cyclohexane et de l'eau.

Le matériau multicouches final est ensuite vulcanisé à 80°C pendant 2 heures.

La structure du matériau final tel que réalisé selon cet exemple est représentée sur le schéma III ci-après :

**SCHÉMA III**

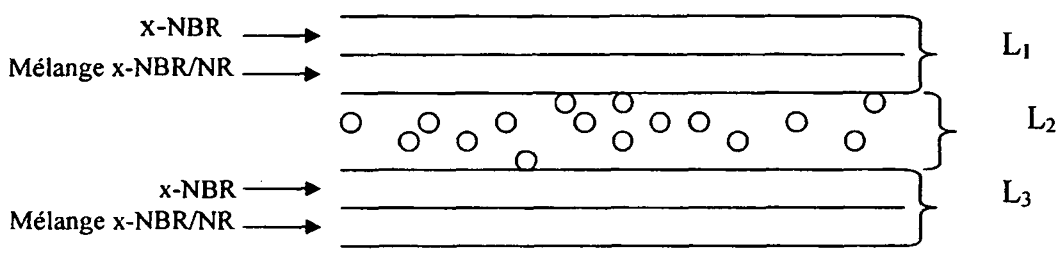


Le matériau multicouches ainsi préparé est ensuite caractérisé selon les méthodes décrites ci-dessus à l'exemple 1.

Les caractéristiques obtenues sont reportées dans le tableau II ci-après :

**Tableau II**

| Couches | Contrainte à la rupture σ (en Mpa) | Constante élastique E (en Mpa) | Produit (σ.e) (en Pa.m) |
|---|---|---|---|
| $L_1$ | 12 | 3 | 3000 |
| $L_2$ | 9[a] | 7 | 1800 |
| $L_3$ | 12 | 3 | 3000 |
| [a] : la valeur indiquée est la contrainte à la rupture de la couche élastomère chargée en chlorure de benzalkonium. | | | |

La quantité de chlorure de benzalkonium déposée sur l'aiguille après le test de perforation selon les conditions décrites ci-dessus à l'exemple 1 est évaluée à 0,6 μg. La vitesse d'expulsion est évaluée à 3 m/s.

**EXEMPLE 3 COMPARATIF: PREPARATION D'UN MATERIAU MULTICOUCHES NON CONFORME A L'INVENTION**

Ce contre exemple illustre la préparation d'un matériau multicouches à base d'élastomères de synthèse de type SEBS mais dont les caractéristiques ne répondent pas à la double inégalité (I) telle que définie selon la présente Invention.

**1) Préparation du matériau multicouche**

Le matériau multicouche est entièrement réalisé à partir de dissolutions de SEBS dans du cyclohexane. Les différents bains d'élastomères utilisés pour réaliser les couches $L_1$ à $L_3$ sont les suivants :

- Couches barrières $L_1$ et $L_3$ : bain d'élastomère constitué par 20 % en poids (par rapport à l'extrait sec) d'un mélange de copolymère SEBS vendu sous la dénomination commerciale Kraton® G1652 par la société Kraton Polymers et d'une huile minérale (Primol® 352, société Esso) utilisée à titre d'agent plastifiant en solution dans du cyclohexane. Au sein de ce mélange les proportions massiques élastomère/agent plastifiant sont 100:30.
- Couche intermédiaire $L_2$ : bain d'élastomère constitué par 20 % en poids (par rapport à l'extrait sec) d'un mélange de copolymère SEBS vendu sous la dénomination commerciale Kraton® G1654 par la société Kraton Polymers et d'une huile minérale (Marcol® 82, société Esso) utilisée à titre d'agent plastifiant en solution dans du cyclohexane. Au sein de ce mélange les proportions massiques élastomère/agent plastifiant sont 100:50. Ce bain d'élastomère contient une dispersion de gouttelettes de substance active (chlorure de benzalkonium en solution dans de l'éthylène glycol, en proportions massiques 1:9) stabilisée par un stabilisant particulaire organique qui est un copolymère dibloc polybutadiène-poly(oxyde d'éthylène) à raison de 5 parts pour 100 parts de solution de chlorure de benzalkonium dans l'éthylène glycol.

Au sein de cette dispersion, le diamètre moyen des gouttelettes (mesuré au microscope optique) est de 30 μm. La fraction volumique des gouttelettes φv est de 0,3 (par rapport à l'extrait sec après mélange avec l'élastomère en solution dans le cyclohexane).

On obtient ainsi un matériau multicouches constitué de deux couches barrières $L_1$ et $L_3$ identiques entres elles, d'une épaisseur de 150 μm chacune et d'une couche intermédiaire $L_2$ d'une épaisseur de 250 μm, renfermant une dispersion de gouttelettes de chlorure de benzalkonium (les épaisseurs sont mesurées au comparateur micrométrique).

Le matériau multicouches ainsi préparé est ensuite caractérisé selon les méthodes décrites ci-dessus à l'exemple 1.

Les caractéristiques obtenues sont reportées dans le tableau III ci-après :

**Tableau III**

| Couches | Contrainte à la rupture σ (en Mpa) | Constante élastique E (en Mpa) | Produit (σ.e) (en Pa.m) |
|---|---|---|---|
| $L_1$ | 25 | 2 | 3775 |
| $L_2$ | 16[a] | 1 | 4000 |
| $L_3$ | 25 | 2 | 3775 |
| [a] : la valeur indiquée est la contrainte à la rupture de la couche élastomère chargée en chlorure de benzalkonium. | | | |

On obtient donc un matériau dont les caractéristiques intrinsèques ne remplissent pas la double inégalité (I) définie précédemment puisque le produit ($\sigma_{2Tot}.e_2$) de la couche intermédiaire $L_2$ est supérieur à celui de chacune des couches barrières $L_1$ et $L_3$.

La quantité de chlorure de benzalkonium déposée sur l'aiguille après le test de perforation selon les conditions décrites ci-dessus à l'exemple 1 est évaluée à 0,1 μg, ce qui correspond à une quantité qui est environ 10 fois inférieure à celle de l'exemple 1. La vitesse d'expulsion est évaluée à 1 m/s, ce qui correspond à une vitesse qui est environ 5 fois moins grande qu'à l'exemple 1.

L'ensemble de ces résultats montre que les matériaux conformes à l'Invention, c'est à dire les matériaux multicouches dans lesquels les caractéristiques intrinsèques de chacune des couches répondent à la double inégalité (I) telle que définie précédemment, présentent des performances améliorées en ce qui concerne la quantité de substance active entrant en contact avec une aiguille en cas de perforation dudit matériau ainsi qu'en ce qui concerne la vitesse à laquelle cette substance active entre en contact avec ladite aiguille.

**Revendications**

1. Matériau élastomère multicouches comportant au moins deux couches barrières externes $L_1$ et $L_3$, présentant respectivement une contrainte à la rupture $\sigma_1$ et $\sigma_3$ et une épaisseur $e_1$ et $e_3$, emprisonnant au moins une couche intermédiaire $L_2$ constituée par une matrice d'élastomère comprenant au moins une dispersion de gouttelettes d'au moins une composition renfermant au moins une substance active, ladite couche intermédiaire $L_2$ ayant une contrainte à la rupture $\sigma_{2Tot}$ et une épaisseur $e_2$, **caractérisé par le fait que** le diamètre moyen desdites gouttelettes est supérieur ou égal à 10 μm et que ledit matériau remplit la double inégalité (I) suivante :

$$(\sigma_{2Tot}.e_2) < (\sigma_1.e_1) \text{ et } (\sigma_{2Tot}.e_2) < (\sigma_3.e_3) \qquad (I)$$

inégalité dans laquelle :

- $\sigma_{2Tot}$ représente la contrainte à la rupture du matériau élastomère chargé constitutif de la couche $L_2$,
- $\sigma_1$, $\sigma_3$, $e_1$, $e_2$ et $e_3$ sont tels que définis ci-dessus.

2. Matériau selon la revendication 1, **caractérisé par le fait que** le produit ($\sigma_{2Tot}.e_2$) répond à la double condition (II) suivante :

$$(\sigma_{2Tot}.e_2) \leq (\sigma_1.e_1)/2 \text{ et } (\sigma_{2Tot}.e_2) \leq (\sigma_3.e_3)/2 \qquad \text{(II)}$$

dans laquelle $\sigma_1$, $\sigma_{2Tot}$, $\sigma_3$, $e_1$, $e_2$ et $e_3$ ont les mêmes significations que celles indiquées à la revendication 1.

3. Matériau selon la revendication 1 ou 2, **caractérisé par le fait que** les contraintes à la rupture $\sigma_1$, $\sigma_2$ et $\sigma_3$ de chacune des couches dudit matériau, identiques ou différentes, varient entre 0,1 et 100 MPa.

4. Matériau selon l'une quelconque des revendications précédentes, **caractérisé par le fait que** les épaisseurs $e_1$, $e_2$ et $e_3$ de chacune des couches dudit matériau, identiques ou différentes, varient entre 25 et 500 μm.

5. Matériau selon l'une quelconque des revendications précédentes, **caractérisé par le fait que** le diamètre moyen desdites gouttelettes est compris entre 10 et 100 μm.

6. Matériau selon l'une quelconque des revendications précédentes, **caractérisé par le fait que** la constante élastique du matériau constitutif de la couche intermédiaire $L_2$ est supérieure à celles des couches barrières $L_1$ et $L_3$ ($E_1$ et $E_3$) soit $E_2 > \max(E_1, E_3)$.

7. Matériau selon la revendication 6, **caractérisé par le fait que** les constantes élastiques de chacune des couches $L_1$, $L_2$ et $L_3$, soit respectivement $E_1$, $E_2$ et $E_3$, sont comprises entre 0,1 et 50 MPa ; les valeurs de $E_1$, et de $E_3$ étant identiques ou différentes.

8. Matériau selon la revendication 7, **caractérisé par le fait que** les constantes élastiques des couches $L_1$ et $L_3$, identiques ou différentes, sont comprises entre 0,1 et 10 MPa, et que la constante élastique de la couche $L_2$ est comprise entre 0,5 et 50 MPa.

9. Matériau selon l'une quelconque des revendications précédentes, **caractérisé par le fait que** le ou les élastomères constituant les couches barrières externes $L_1$ et $L_3$, ainsi que la couche intermédiaire $L_2$, sont choisis parmi le caoutchouc naturel, le polybutadiène, le polyisoprène, le polychloroprène, le polyuréthane, les copolymères ou polymères acryliques, les élastomères silicones, les copolymères SBR *(Styrene Butadiene Rubber),* SBS *(Styrene Butadiene Styrene),* isobutène-isoprène tels que le caoutchouc butyle, NBR *(Nitrile Butadiene Rubber),* x-NBR (carboxylated Nitrile Butadiene Rubber), SIS *(Styrene Isoprene Styrene)* ou **SEBS** *(Styrene Ethylene Butylene Styrene)* et leurs mélanges.

10. Matériau selon la revendication 9, **caractérisé par le fait que** lesdits élastomères sont choisis parmi le SIS *(Styrene Isoprene Styrene)* et le SEBS *(Styrene Ethylene Butylene Styrene).*

11. Matériau selon l'une quelconque des revendications précédentes, **caractérisé par le fait que** l'une au moins des couches barrières $L_1$ et $L_3$, et/ou la couche intermédiaire $L_2$ contient, en outre, un ou plusieurs agent(s) plastifiant (s) ou flexibilisant(s).

12. Matériau selon la revendication 11, **caractérisé par le fait que** le ou les agents plastifiants représentent de 5 à 500 parts pour 100 parts d'élastomère constituant la couche au sein de laquelle ils sont présents.

13. Matériau selon l'une quelconque des revendications précédentes, **caractérisé par le fait que** chaque couche $L_1$ ou $L_3$ résulte de la superposition de deux ou plusieurs sous-couches de nature chimique équivalente ou non.

14. Matériau selon l'une quelconque des revendications précédentes, **caractérisé par le fait que** la substance chimique active est choisie parmi les agents anti-corrosion, les lubrifiants, les marqueurs chimiques, les produits à changement de phase, les ralentisseurs de particules énergétiques (radiation), les agents possédant un pouvoir désinfectant, les agents odorants ou hydratants, les colorants détecteurs de coupures, les particules métalliques, et leurs mélanges.

15. Matériau selon la revendication 14, **caractérisé par le fait que** la substance chimique active est choisie les biocides, les biguanides, le phtalaldéhyde, les dérivés phénoliques, le formol, les tensioactifs nonioniques comportant au moins une séquence polyoxyéthylène, l'hexamidine, les composés iodés de polyvinylpyrrolidone, les tensioactifs nonioniques à activité virucide, les bichromates et hypochlorites de sodium et de potassium, et leurs mélanges.

**16.** Matériau selon l'une quelconque des revendications précédentes, **caractérisé par le fait que** la composition sous forme de gouttelettes renferme en outre un ou plusieurs diluants permettant de solubiliser ladite ou lesdites substances chimiques actives.

**17.** Matériau selon l'une quelconque des revendications précédentes, **caractérisé par le fait que** la composition sous forme de gouttelettes est sous forme liquide, gélifiée ou contient des parties cristallines.

**18.** Matériau selon l'une quelconque des revendications précédentes, **caractérisé par le fait que** la couche intermédiaire $L_2$ est formée d'une superposition de deux ou plusieurs sous-couches intermédiaires comprenant chacune une dispersion de gouttelettes, la nature des substances actives contenues dans chacune desdites sous-couches étant identiques ou différentes d'une sous-couche à une autre.

**19.** Matériau selon l'une quelconque des revendications 1 à 17, **caractérisé par le fait que** la couche intermédiaire $L_2$ est formée par une seule couche renfermant une dispersion de gouttelettes contenant des substances chimiques actives différentes d'une gouttelette à une autre.

**20.** Matériau selon l'une quelconque des revendications précédentes, **caractérisé par le fait qu**'il se présente sous la forme de gants, de doigtiers, de préservatifs, de bandes ou de pansements.

**21.** Utilisation d'au moins un matériau tel que défini à l'une quelconque des revendications 1 à 19, pour la fabrication d'articles élastomères de protection.

**22.** Utilisation d'au moins un matériau tel que défini à l'une quelconque des revendications 1 à 19, pour la fabrication de gants, de doigtiers, de préservatifs, de bandes ou de pansements.

**Claims**

**1.** Multilayer elastomeric material comprising at least two outer barrier layers $L_1$ and $L_3$, respectively having a breaking stress $\sigma_1$ and $\sigma_3$ and a thickness $e_1$ and $e_3$, enclosing at least one intermediate layer $L_2$ consisting of an elastomeric matrix comprising at least one dispersion of droplets of at least one composition containing at least one active substance, the said intermediate layer $L_2$ having a breaking stress $\sigma_{2Tot}$ and a thickness $e_2$, **characterized in that** the mean diameter of the said droplets is greater than or equal to 10 $\mu$m and that the said material satisfies the following double inequality (I):

$$(\sigma_{2Tot}.e_2) < (\sigma_1.e_1) \text{ and } (\sigma_{2Tot}.e_2) < (\sigma_3.e_3) \qquad (I)$$

in which inequality:

- $\sigma_{2Tot}$ represents the breaking stress of the charged elastomeric material constituting the layer $L_2$,
- $\sigma_1$, $\sigma_3$, $e_1$, $e_2$ and $e_3$ are as defined above.

**2.** Material according to Claim 1, **characterized in that** the product $(\sigma_{2Tot}.e_2)$ corresponds to the following double condition (II):

$$(\sigma_{2Tot}.e_2) \leq (\sigma_1.e_1)/2 \text{ and } (\sigma_{2Tot}.e_2) \leq (\sigma_3.e_3)/2 \qquad (II)$$

in which $\sigma_1$, $\sigma_{2Tot}$, $\sigma_3$, $e_1$, $e_2$ and $e_3$ have the same meanings as those given in Claim 1.

**3.** Material according to Claim 1 or 2, **characterized in that** the breaking stresses $\sigma_1$, $\sigma_2$ and $\sigma_3$ of each of the layers of the said material, which may be identical or different, range between 0.1 and 100 MPa.

**4.** Material according to any one of the preceding claims, **characterized in that** the thicknesses $e_1$, $e_2$ and $e_3$ of each of the layers of the said material, which may be identical or different, range between 25 and 500 $\mu$m.

**5.** Material according to any one of the preceding claims, **characterized in that** the mean diameter of the said droplets is between 10 and 100 μm.

**6.** Material according to any one of the preceding claims, **characterized in that** the elastic constant of the constituent material of the intermediate layer $L_2$ is greater than those of the barrier layers $L_1$ and $L_3$ ($E_1$ and $E_3$), i.e. $E_2 > max(E_1, E_3)$.

**7.** Material according to Claim 6, **characterized in that** the elastic constants of each of the layers $L_1$, $L_2$ and $L_3$, i.e. $E_1$, $E_2$ and $E_3$, respectively, are between 0.1 and 50 MPa; the values of $E_1$ and $E_3$ being identical or different.

**8.** Material according to Claim 7, **characterized in that** the elastic constants of the layers $L_1$ and $L_3$, which may be identical or different, are between 0.1 and 10 MPa and the elastic constant of the layer $L_2$ is between 0.5 and 50 MPa.

**9.** Material according to any one of the preceding claims, **characterized in that** the elastomer(s) constituting the outer barrier layers $L_1$ and $L_3$ and also the intermediate layer $L_2$ are chosen from natural rubber, polybutadiene, polyisoprene, polychloroprene, polyurethane, acrylic polymers or copolymers, silicone elastomers, SBR *(Styrene Butadiene Rubber)* copolymers, SBS *(Styrene Butadiene Styrene)* copolymers, isobutene-isoprene copolymers such as butyl rubber, NBR *(Nitrile Butadiene Rubber)* copolymers, x-NBR (carboxylated Nitrile Butadiene Rubber) copolymers, SIS *(Styrene Isoprene Styrene)* copolymers or SEBS *(Styrene Ethylene Butylene Styrene)* copolymers and blends thereof.

**10.** Material according to Claim 9, **characterized in that** the said elastomers are chosen from SIS *(Styrene Isoprene Styrene)* and SEBS *(Styrene Ethylene Butylene Styrene).*

**11.** Material according to any one of the preceding claims, **characterized in that** at least one of the barrier layers $L_1$ and $L_3$, and/or the intermediate layer $L_2$, also contains one or more plasticizer(s) or flexibilizer(s).

**12.** Material according to Claim 11, **characterized in that** the plasticizer(s) represent(s) from 5 to 500 parts per 100 parts of elastomer constituting the layer in which they are present.

**13.** Material according to any one of the preceding claims, **characterized in that** each layer $L_1$ or $L_3$ results from the superposition of two or more sublayers of equivalent or non-equivalent chemical nature.

**14.** Material according to any one of the preceding claims, **characterized in that** the active chemical substance is chosen from anticorrosion agents, lubricants, chemical markers, phase-change products, energetic-particle (radiation) decelerators, agents with disinfecting power, odoriferous agents or moisturizers, dyes for detecting cuts, metallic particles, and mixtures thereof.

**15.** Material according to Claim 14, **characterized in that** the active chemical substance is chosen from biocides, biguanides, phthalaldehyde, phenolic derivatives, formaldehyde, nonionic surfactants comprising at least one polyoxyethylene sequence, hexamidine, iodinated polyvinylpyrrolidone compounds, nonionic surfactants with virucidal activity, sodium and potassium dichromates and hypochlorites, and mixtures thereof.

**16.** Material according to any one of the preceding claims, **characterized in that** the composition in the form of droplets also contains one or more diluents for dissolving the said active chemical substance(s).

**17.** Material according to any one of the preceding claims, **characterized in that** the composition in the form of droplets is in liquid, gelled form or contains crystalline parts.

**18.** Material according to any one of the preceding claims, **characterized in that** the intermediate layer $L_2$ is formed from a superposition of two or more intermediate sublayers each comprising a dispersion of droplets, the nature of the active substances contained in each of the said sublayers being identical or different from one sublayer to another.

**19.** Material according to any of Claims 1 to 17, **characterized in that** the intermediate layer $L_2$ is formed by a single layer containing a dispersion of droplets containing active chemical substances that are different from one droplet to another.

**20.** Material according to any one of the preceding claims, **characterized in that** it is in the form of gloves, finger stalls,

condoms, tapes or dressings.

**21.** Use of at least one material as defined in any one of Claims 1 to 19, for the manufacture of protective elastomeric articles.

**22.** Use of at least one material as defined in any one of Claims 1 to 19, for the manufacture of gloves, finger stalls, condoms, tapes or dressings.

## Patentansprüche

**1.** Mehrschichtiges Elastomermaterial, umfassend wenigstens zwei äußere Sperrschichten $L_1$ und $L_3$, die jeweils eine Bruchbeanspruchung $\sigma_1$ und $\sigma_3$ und eine Dicke $e_1$ und $e_3$ aufweisen, die wenigstens eine Zwischenschicht $L_2$ einschließen, welche durch eine Elastomermatrix gebildet wird, die wenigstens eine Dispersion von Tröpfchen wenigstens einer Zusammensetzung, die wenigstens eine aktive Substanz enthält, umfasst, wobei die Zwischenschicht $L_2$ eine Bruchbeanspruchung $\sigma_{2Tot}$ und eine Dicke $e_2$ hat, **dadurch gekennzeichnet, dass** der mittlere Durchmesser der Tröpfchen über oder gleich 10 µm ist und dass das Material die folgende doppelte Ungleichung (I) erfüllt:

$$(\sigma_{2Tot} \cdot e_2) < (\sigma_1 \cdot e_1) \text{ und } (\sigma_{2Tot} \cdot e_2) < (\sigma_3 \cdot e_3)$$

wobei in der Ungleichung:

- $\alpha_{2Tot}$ die Bruchbeanspruchung des beladenen Elastomermaterials, das Bestandteil der Schicht $L_2$ ist, darstellt,
- $\sigma_1$, $\sigma_3$, $e_1$, $e_2$ und $e_3$ wie oben definiert sind.

**2.** Material nach Anspruch 1, **dadurch gekennzeichnet, dass** das Produkt ($\sigma_{2Tot}.e_2$) der folgenden zweifachen Bedingung (II) entspricht:

$$(\sigma_{2Tot} \cdot e_2) \leq (\sigma_1 \cdot e_1)/2 \text{ und } (\sigma_{2Tot} \cdot e_2) \leq (\sigma_3 \cdot e_3)/2$$

wobei $\sigma_1$, $\sigma_{2Tot}$, $\sigma_3$, $e_1$, $e_2$ und $e_3$ die selben Bedeutungen, wie in Anspruch 1 angegeben, haben.

**3.** Material nach Anspruch 1 oder 2, **dadurch gekennzeichnet, dass** die Bruchbeanspruchungen $\sigma_1$, $\sigma_2$ und $\sigma_3$ von jeder der Schichten des Materials, die identisch oder unterschiedlich sind, zwischen 0,1 und 100 MPa variieren.

**4.** Material nach einem der vorangehenden Ansprüche, **dadurch gekennzeichnet, dass** die Dicken $e_1$, $e_2$ und $e_3$ von jeder der Schichten des Materials, die identisch oder unterschiedlich sind, zwischen 25 und 500 µm variieren.

**5.** Material nach einem der vorangehenden Ansprüche, **dadurch gekennzeichnet, dass** der mittlere Durchmesser der Tröpfchen zwischen 10 und 100 µm liegt.

**6.** Material nach einem der vorangehenden Ansprüche, **dadurch gekennzeichnet, dass** die Elastizitätskonstante des Materials, das Bestandteil der Zwischenschicht $L_2$ ist, über denjenigen der Sperrschichten $L_1$ und $L_3$ ($E_1$ und $E_3$) liegt, also $E_2$>max ($E_1$, $E_3$).

**7.** Material nach Anspruch 6, **dadurch gekennzeichnet, dass** die Elastizitätskonstanten von jeder der Schichten $L_1$, $L_2$ und $L_3$, nämlich $E_1$, $E_2$ bzw. $E_3$, zwischen 0, 1 und 50 MPa liegen, wobei die Werte von $E_1$ und $E_3$ identisch oder unterschiedlich sind.

**8.** Material nach Anspruch 7, **dadurch gekennzeichnet, dass** die Elastizitätskonstanten der Schichten $L_1$ und $L_3$, die identisch oder unterschiedlich sind, zwischen 0,1 und 10 MPa liegen und dass die Elastizitätskonstante der Schicht $L_2$ zwischen 0,5 und 50 MPa liegt.

**9.** Material nach einem der vorangehenden Ansprüche, **dadurch gekennzeichnet, dass** das Elastomer oder die Elastomeren, das/die die äußeren Sperrschichten $L_1$ und $L_3$ sowie die Zwischenschicht $L_2$ bildet/bilden, unter Naturkautschuk, Polybutadien, Polyisopren, Polychloropren, Polyurethan, Acryl-Copolymeren oder -Polymeren, Silikonelastomeren, SBR (Styrol-Butadien-Kautschuk)-, SBS (Styrol-Butadien-Styrol)-, Isobuten-Isopren-, wie Butylkautschuk, NBR (Nitril-Butadien-Kautschuk)-, x-NBR (carboxylierter Nitril-Butadien-Kautschuk)-, SIS (Styrol-Isopren-Styrol)- oder SEBS (Styrol-Ethylen-Butylen-Styrol)-Copolymeren und ihren Gemischen ausgewählt sind.

**10.** Material nach Anspruch 9, **dadurch gekennzeichnet, dass** die Elastomeren aus SIS (Styrol-Isopren-Styrol) und SEBS (Styrol-Ethylen-Butylen-Styrol) ausgewählt sind.

**11.** Material nach einem der vorangehenden Ansprüche, **dadurch gekennzeichnet, dass** wenigstens eine der Sperrschichten $L_1$ und $L_3$ und/oder die Zwischenschicht $L_2$ ausserdem einen oder mehrere Weichmacher oder Plastikatoren enthält.

**12.** Material nach Anspruch 11, **dadurch gekennzeichnet, dass** der Weichmacher oder die Weichmacher 5 bis 500 Teile pro 100 Teile Elastomer, das die Schicht bildet, in der sie vorliegen, darstellt/darstellen.

**13.** Material nach einem der vorangehenden Ansprüche, **dadurch gekennzeichnet, dass** jede Schicht $L_1$ oder $L_3$ aus der Übereinanderlagerung von zwei oder mehr Unterschichten mit äquivalenter oder nicht-äquivalenter chemischer Natur resultiert.

**14.** Material nach einem der vorangehenden Ansprüche, **dadurch gekennzeichnet, dass** die aktive chemische Substanz ausgewählt ist aus Antikorrosionsmitteln, Schmiermitteln, chemischen Markern, Phasentransferprodukten, Mitteln zur Verlangsamung von energetischen Teilchen (Strahlung), Mitteln, die Desinfektionswirkung besitzen, duftenden oder hydratisierenden Mitteln, Färbemitteln, die Spaltungsdetektoren sind, metallischen Partikeln und deren Gemischen.

**15.** Material nach Anspruch 14, **dadurch gekennzeichnet, dass** die aktive chemische Substanz ausgewählt ist aus Bioziden, Biguaniden, Phthalaldehyd, Phenolderivaten, Formol, nicht-ionischen oberflächenaktiven Mitteln, die wenigstens eine Polyoxyethylensequenz umfassen, Hexamidin, iodierten Polyvinylpyrrolidon-Verbindungen, nicht-ionischen oberflächenaktiven Mitteln mit Virozidaktivität, Bichromaten und Hypochloriten von Natrium und Kalium und deren Gemischen.

**16.** Material nach einem der vorangehenden Ansprüche, **dadurch gekennzeichnet, dass** die Zusammensetzung in Form von Tröpfchen außerdem ein Verdünnungsmittel oder mehrere Verdünnungsmittel umfasst, das/die es ermöglicht/ermöglichen, die aktive chemische Substanz oder die aktiven chemischen Substanzen zu solubilisieren.

**17.** Material nach einem der vorangehenden Ansprüche, **dadurch gekennzeichnet, dass** die Zusammensetzung in Form von Tröpfchen in flüssiger, gelierter Form ist oder kristalline Teile enthält.

**18.** Material nach einem der vorangehenden Ansprüche, **dadurch gekennzeichnet, dass** die Zwischenschicht $L_2$ durch Überlagerung von zwei oder mehreren Zwischenunterschichten gebildet wird, von denen jede eine Dispersion von Tröpfchen umfasst, wobei die Natur der aktiven Substanzen, die in jeder der Unterschichten enthalten sind, von einer Unterschicht zur anderen identisch oder unterschiedlich ist.

**19.** Material nach einem der Ansprüche 1 bis 17, **dadurch gekennzeichnet, dass** die Zwischenschicht $L_2$ durch eine einzige Schicht gebildet wird, die eine Dispersion von Tröpfchen umfasst, die aktive chemische Substanzen enthalten, die von einem Tröpfchen zum anderen unterschiedlich sind.

**20.** Material nach einem der vorangehenden Ansprüche, **dadurch gekennzeichnet, dass** es in Form von Handschuhen, Fingerlingen, Präservativen, Bändern oder Verbänden vorliegt.

**21.** Verwendung wenigstens eines Materials, wie es in einem der Ansprüche 1 bis 19 definiert ist, für die Herstellung von elastomeren Schutzgegenständen.

**22.** Verwendung wenigstens eines Materials, wie es in einem der Ansprüche 1 bis 19 definiert ist, für die Herstellung von Handschuhen, Fingerlingen, Präservativen, Bändern oder Verbänden.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0981573 A
- EP 771837 A
- EP 981573 A